(19) Europäisches Patentamt / European Patent Office / Office européen des brevets

(11) **EP 2 702 878 B1**

(12) **EUROPEAN PATENT SPECIFICATION**

(45) Date of publication and mention
of the grant of the patent:
**13.01.2016 Bulletin 2016/02**

(21) Application number: **12776449.6**

(22) Date of filing: **26.04.2012**

(51) Int Cl.:
*A23L 2/39* (2006.01)     *A23L 2/00* (2006.01)
*A61K 8/44* (2006.01)     *A61K 8/67* (2006.01)
*A61K 9/08* (2006.01)     *A61K 9/14* (2006.01)
*A61K 31/198* (2006.01)     *A61K 31/375* (2006.01)
*A61P 3/02* (2006.01)     *A61Q 19/00* (2006.01)
*B65D 51/28* (2006.01)

(86) International application number:
**PCT/JP2012/002854**

(87) International publication number:
**WO 2012/147351 (01.11.2012 Gazette 2012/44)**

(54) **USE OF A POWDERED RAW MATERIAL, BOTTLE CAP, AND CONTAINER COMPRISING SAID BOTTLE CAP**

VERWENDUNG EINES PULVERFÖRMIGES ROHMATERIAL, FLASCHENVERSCHLUSS UND BEHÄLTER MIT DEM FLASCHENVERSCHLUSS

UTILISATION D'UN MATÉRIAU DE DÉPART PULVÉRULENT, BOUCHON DE FLACON ET RÉCIPIENT ÉQUIPÉ DUDIT BOUCHON DE FLACON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2011 JP 2011101437**

(43) Date of publication of application:
**05.03.2014 Bulletin 2014/10**

(73) Proprietor: **Fresh Co., Ltd.**
**Sakata-city**
**Yamagata 999-8201 (JP)**

(72) Inventor: **SUZUKI, Yoshihiro**
**Sakata-City**
**Yamagata 999-8201 (JP)**

(74) Representative: **Hoffmann Eitle**
**Patent- und Rechtsanwälte PartmbB**
**Arabellastraße 30**
**81925 München (DE)**

(56) References cited:
WO-A1-2010/023933     WO-A1-2010/023933
JP-A- 5 124 934     JP-A- 5 124 934
JP-A- 2002 125 593     JP-A- 2005 287 469
JP-A- 2005 287 469

**Description**

TECHNICAL FIELD

The present invention relates to a use of a powdered raw material, a bottle cap and a container with the bottle cap.

BACKGROUND ART

A type of a bottle cap which has a housing portion that houses a powdered raw material, and can be opened by an uncapping operation, and then causes the raw material to be released into the bottle is known as a bottle cap which closes an opening portion of a bottle filled with a liquid (refer to Patent Document 1).
In such a bottle cap, the housing portion can be opened by the uncapping operation, and the powdered raw material which is housed in the housing portion is released into the bottle which is filled with the liquid. Then, the powdered raw material released is dispersed into the liquid in the bottle, and is suspended or dissolved.
Patent Document 1 describes that since the powdered raw material and the liquid are mixed just before drinking when using such a bottle cap containing the powdered raw material, the deterioration over a lapse of time in the bottle is small and thus it is possible to enjoy beverage while maintaining a fresh state with excellent taste and flavor.

RELATED DOCUMENT

PATENT DOCUMENT

[Patent Document 1] Japanese Unexamined Patent Publication No.2003-34367
[Patent Document 2] Japanese Unexamined Patent Publication No.2006-296341

DISCLOSURE OF THE INVENTION

However, when a powdered raw material which is filled in a bottle cap is released into a bottle, there is a case where the powdered raw material clumps together and forms lumps in a liquid. Since, when there are lumps during drinking, the sensation felt on the tongue and the throat is unpleasant, the drinking feeling is bad.
On the other hand, the powdered raw material for a beverage as described in Patent Document 2 is known to have an excellent dispersibility in the liquid. However, when such a powdered raw material is used as it is in the bottle cap described above, there is a case where the powdered raw material is not filled well in a housing portion of the bottle cap, and is scattered to the outside of the bottle cap. Thus, the powdered raw materials for the beverages in the related art were difficult to use in filling the bottle cap.
The present invention was made in view of the problems described above, and provides the powdered raw material is excellent in fillability in the bottle cap and dispersibility in the liquid.
According to the present invention, there is provided a use of a powdered raw material with the features of claim 1.
Furthermore, according to the present invention, there is provided a bottle cap with the features of claim 13.
Furthermore, according to the present invention, a container which is provided with the bottle cap and the bottle to which the bottle cap is mounted is provided.
According to the present invention, the powdered raw material of which the degree of dispersion is equal to or higher than 5% is used as the powdered raw material which is housed in the bottle cap. When being released into the bottle which is filled with the liquid, the powdered raw material of which the degree of dispersion is equal to or higher than 5% is dispersed without clumping together and forming lumps in the liquid. As a result, it is possible to obtain the beverage of which the sensation felt on the tongue and the throat is excellent and the drinking feeling is excellent.
Furthermore, according to the present invention, the powdered raw material of which the degree of dispersion is equal to or lower than 40% is used as the powdered raw material which is housed in the bottle cap. When being filled in the housing portion of the bottle cap, the powdered raw material of which the degree of dispersion is equal to or lower than 40% may be filled without being scattered to the outside of the bottle cap. As a result, it is possible to stably produce with high yield the bottle cap which is filled with the powder.
According to the present invention, it is possible to provide the powdered raw material which is excellent in fillability in the bottle cap and dispersibility in the liquid.

BRIEF DESCRIPTION OF THE DRAWINGS

The above-described object and other objects, features and advantages become further apparent from preferred embodiments which will be described below and the following drawings accompanied therewith.

FIG. 1 shows a front view illustrating a container according to an embodiment.
FIG. 2 shows an exploded cross-sectional view of a bottle cap according to the embodiment.
FIG. 3 shows a cross-sectional view illustrating a state at which a powdered raw material is housed in the bottle cap according to the embodiment.
FIG. 4 shows a cross-sectional view illustrating a state at which the bottle cap according to the embodiment is uncapped.

DESCRIPTION OF EMBODIMENTS

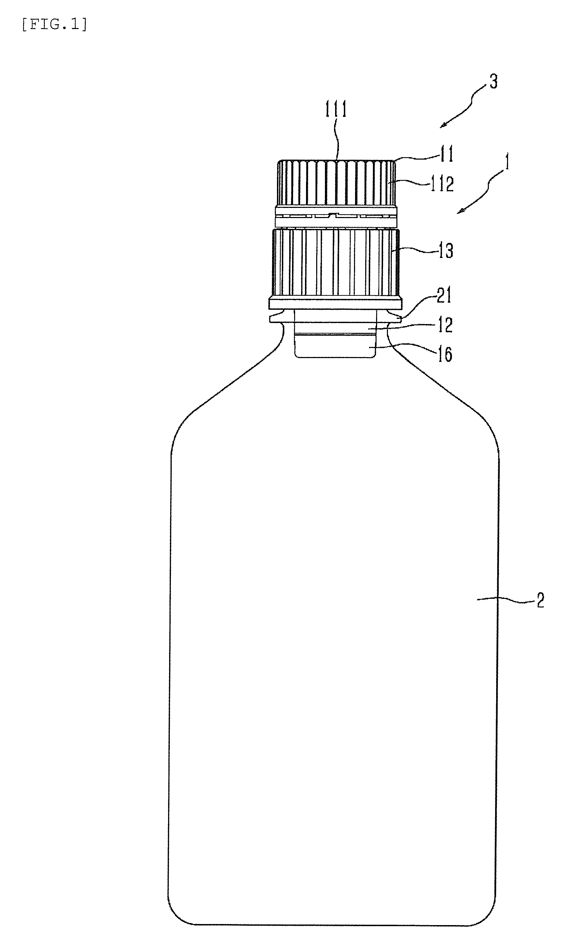

Hereinafter, embodiments of the present invention will be described with reference to FIGS. 1 to 4. Moreover, the same components are represented as common reference numerals, and the description thereof will not be repeated.

(Powdered Raw Material)

First, a powdered raw material according to an embodiment is described.

The powdered raw material according to the embodiment is a powdered raw material which is housed in a housing portion 12 of a type of a bottle cap 1 which has the housing portion 12 in which the powdered raw material is housed, and of which the housing portion 12 is opened by an uncapping operation, and then causes the powdered raw material to be released into the bottle.

The shape of the powdered raw material, is not particularly limited, and may be spherical, massive, flaky, plate-like shape or the like. Among these, the spherical shape is preferable.

Using a multi-functional powder property measuring instrument multi-tester, the powdered raw material according to the embodiment is dropped onto a watch glass from a powder falling point, a dropped mass and a mass on the watch glass are measured, and a degree of dispersion which is obtained from the following formula (1) is equal to or higher than 5%, is preferably equal to or higher than 8%, and is more preferably equal to or higher than 10%.

$$\text{degree of dispersion } [\%] = (\text{dropped mass} - \text{mass on the watch glass}) / \text{dropped mass} \times 100 \quad (1)$$

In a case where the degree of dispersion is equal to or higher than the above-described values, when being released into the bottle which is filled with a liquid, the powdered raw material is dispersed without being aggregated and being lumped in the liquid. As a result, it is possible to obtain a beverage of which the sensation felt on the tongue and the throat is excellent, and the drinking feeling is excellent.

Furthermore, the degree of dispersion of the powdered raw material according to the embodiment is equal to or lower than 40%, is preferably equal to or lower than 35%, and is more preferably equal to or lower than 30%. In a case where the degree of dispersion is equal to or lower than the above-described values, when filled in the housing portion 12 of the bottle cap, the powdered raw material may be filled without being scattered to the outside of the bottle cap. As a result, it is possible to stably produce with high yield the bottle cap which is filled with the powder.

A degree of compaction, of the powdered raw material according to the embodiment, which is calculated by the following formula (2) is preferably equal to or higher than 30%, and is more preferably equal to or higher than 40%. In a case where the degree of compaction is equal to or higher than the above-described values, it is possible to suppress a fluidity of the powdered raw material, and further improve the fillability in the housing portion 12 of the bottle cap.

$$\text{degree of compaction } [\%] = (\text{compact bulk density} - \text{loose bulk density}) / \text{compact bulk density} \times 100 \quad (2)$$

Moreover, the loose bulk density means a bulk density in a state of loose packing, and the compact bulk density means a bulk density in a case where a tapping treatment in the measurement of the loose bulk density is performed and tight packing is performed.

Furthermore, the degree of compaction of the powdered raw material according to the embodiment is preferably equal to or lower than 60%, and more preferably equal to or lower than 50%. In a case where the degree of compaction is equal to or lower than the above-described values, the liquid can easily penetrate into the inside of a mass of the powdered raw material. As a result, it is possible to further suppress a generation of lumps of the powdered raw material in the liquid. Additionally, in a case where the degree of compaction is equal to or lower than the above-described values, a dispersibility at the time of release in the liquid is further excellent since a consolidation of the powdered raw material which is housed in the bottle cap is unlikely to occur.

The loose bulk density of the powdered raw material according to the embodiment is preferably equal to or higher than 0.1 $g/cm^3$, and is more preferably equal to or higher than 0.2 $g/cm^3$. In a case where the loose bulk density is equal to or higher than the above-described values, it is possible to further increase a housing capacity of the powdered raw material in the housing portion 12.

Furthermore, the loose bulk density is preferably equal to or lower than 0.4 $g/cm^3$, and is more preferably equal to or lower than 0.3 $g/cm^3$. In a case where the loose bulk density is equal to or lower than the above-described values, the dispersibility at the time of release in the liquid is further excellent since the consolidation of the powdered raw material which is housed in the bottle cap is unlikely to occur.

The compact bulk density of the powdered raw material according to the embodiment is preferably equal to or higher than 0.2 $g/cm^3$, and is more preferably equal to or higher than 0.3 $g/cm^3$. In a case where the compact bulk density is equal to or higher than the above-described values, it is possible to further increase the housing capacity of the powdered raw material in the housing portion 12.

Furthermore, the compact bulk density is preferably equal to or lower than 0.8 $g/cm^3$ and is more preferably equal to or lower than 0.6 $g/cm^3$. In a case where the compact bulk density is equal to or lower than the above-described values, it is possible to further improve the fluidity of the powdered raw material, and further suppress a clumping of the powdered raw material in the liquid.

A particle diameter $D_{10}$ of the powdered raw material according to the embodiment is preferably equal to or higher than 1 $\mu$m, and is more preferably equal to or higher than 2 $\mu$m. Here, $D_x$ represents the particle diameter of which a cumulative mass distribution becomes X% in a particle size distribution measured using a laser diffraction-scattering particle size distribution analyzer.

In a case where the particle diameter $D_{10}$ of the powdered raw material is equal to or higher than the above-described values, it is possible to further improve the fluidity of the powdered raw material, and further suppress the coagulation of the powdered raw material in the liquid.

Additionally, in a case where the particle diameter $D_{10}$ of the powdered raw material is equal to or higher than the above-described values, the dispersibility at the time of release in the liquid is further excellent since the consolidation of the powdered raw material which is housed in the bottle cap is unlikely to occur.

The particle diameter $D_{90}$ of the powdered raw material according to the embodiment is preferably equal to or lower than 50 $\mu$m, and is more preferably equal to or lower than 40 $\mu$m. In a case where the particle diameter $D_{90}$ is equal to or lower than the above-described values, it is possible to further increase the housing capacity of the powdered raw material in the housing portion 12. Furthermore, it is possible to further suppress the generation of lumps which is resultant from powder having a large particle diameter.

The particle diameter $D_{50}$ of the powdered raw material according to the embodiment is preferably equal to or higher than 3 $\mu$m, and is more preferably equal to or higher than 5 $\mu$m. In a case where the particle diameter $D_{50}$ is equal to or higher than the above-described values, the dispersibility in the liquid is further improved since it is possible to further suppress the coagulation of the powdered raw material in the liquid. Additionally, in a case where the particle diameter $D_{50}$ is equal to or higher than the above-described values, the fillability of the powdered raw material in the housing portion 12 is further improved since the consolidation of the powdered raw material which is housed in the bottle cap is unlikely to occur.

In addition, the particle diameter $D_{50}$ is preferably equal to or lower than 15 $\mu$m, and is more preferably equal to or lower than 12 $\mu$m. In a case where the particle diameter $D_{50}$ is equal to or lower than the above-described values, it is possible to further increase the housing capacity of the powdered raw material in the housing portion 12. Furthermore, it is possible to further suppress the generation of lumps which is derived from powder having a large particle diameter.

A standard deviation sigma of the particle size distribution of the powdered raw material according to the embodiment is preferably equal to or lower than 15 $\mu$m and is more preferably equal to or lower than 12 $\mu$m. In a case where the standard deviation sigma of the particle size distribution is equal to or lower than the above-described values, it is possible to further improve the fluidity of the powdered raw material, and further suppress the coagulation of the powdered raw material in the liquid. Additionally, in a case where the standard deviation sigma of the particle size distribution is equal to or lower than the above-described values, the fillability of the powdered raw material in the housing portion 12 is further improved since the consolidation of the powdered raw material which is housed in the bottle cap is unlikely to occur. Furthermore, it is possible to further suppress the generation of lumps which is derived from powder having a large particle diameter.

A uniformity, which is defined by a particle diameter $D_{60}$/particle diameter $D_{10}$, of the powdered raw material according to the embodiment, is preferably equal to or lower than 5 and more preferably equal to or lower than 4. In a case where the uniformity is equal to or lower than the above-described values, it is possible to further improve the fluidity of the powdered raw material and further suppress the coagulation of the powdered raw material in the liquid.

Additionally, in a case where the uniformity is equal to or lower than the above-described values, the fillability of the powdered raw material in the housing portion 12 is further improved since the consolidation of the powdered raw material which is housed in the bottle cap is unlikely to occur.

(Method for Preparation of the Powdered Raw Material)

A method for preparation of the powdered raw material which has a powder property described above according to an embodiment is not particularly limited, and for example, may be obtained by pulverizing coarse particles of the powdered raw material using a mechanical pulverization method and, then, adjusting the particle diameter by a classification operation or a granulation operation.

As a mechanical pulverization method of the above, the methods are not particularly limited, and well-known methods have been adopted. For example, methods using a stone mortar, a ball mill, a jet mill, an airflow type pulverizer or the like are exemplified.

Well-known methods in the related art are adopted as the classification operation described above, and are not particularly limited. For example, a method which classifies using a screening machine, a method which classifies using a gravity classification, a method which classifies using a centrifuge, and a method which classifies using an inertial classification and the like are exemplified.

Well-known methods in the related art are adopted as the granulation operation described above, and are not particularly limited. For example, a fluidized bed granulation, a dynamic rolling granulation, a spray drying, a coating granulation, a stirring granulation, a drying-compression granulation, an extrusion granulation and the like are exemplified.

A specific powdered raw material according to the embodiment is not particularly limited. Powdered raw materials which are used in supplements, foods and beverage, pharmaceuticals, cosmetics and the like are preferably used. Particularly, the powdered raw materials which are used in supplements, foods and beverages and the like are preferably used.

As the powdered raw material, for example: vitamins such as vitamin A, vitamin B group, vitamin C, vitamin E, vitamin D, and vitamin K; minerals such as zinc, iron, calcium, magnesium, sodium, potassium, and phosphorus; amino acids such as tryptophan, threonine, leucine, isoleucine, lysine, and methionine; fatty acids such as alpha-linolenic acid, eicosapentaenoic acid, docosahexaenoic acid and arachidonic acid; dietary fibers such as pectin, agarose, glucomannan, and sodium alginate; sugars such as starch, cellulose, chitin, chitosan, hyaluronic acid, and oligosaccharides; proteins such as casein and collagen; tumerics such as spring termeric, fall turmeric and purple turmeric; polyphenols such as catechin, isoflavone, and tannin; fungus powders such as lactic acid bacteria powder and bifidobacteria powder, enzyme powder, tea raw materials such as powdered green tea, green tea, oolong tea, black tea, brown rice tea, barley tea, adlay tea, and hub tea; fruit juice powders such as cassis, orange, lemon, blueberry, acerola, banana, and raspberry; vegetable powders such as vegetable juice drink, favorite beverage powder such as coffee, and cocoa; yeast powder; and the like are exemplified. Among them, particularly, tea raw materials such as green tea, lactic acid bacteria powder, vitamins, and yeast powder are preferably used.

Moreover, the active raw materials such as enzymes, bacteria, and yeast are not particularly limited. For example, it is possible to prepare those raw materials into a powder shape while maintaining their activity by frost-shattering the raw materials thereof.

(Bottle Cap)

Hereinafter, an summary of the bottle cap 1 according to the embodiment will be described with reference to FIGS. 1 to 4.

The bottle cap 1 includes: an inner cylinder portion 10 which has a cylindrical housing portion 12 of which one end face is closed and the other end face is open and which is inserted into an opening portion 21 of a bottle 2; an outer cylinder portion 15 having an outer cylinder portion body 13 into which the housing portion 12 is inserted, and of which the one end portion is screwed into the inner cylinder portion 10 and the other end portion is inserted into the opening portion 21 of the bottle 2, being screwed into the opening portion 21 of the bottle 2; and a bottom lid portion 16 which closes the opening of the housing portion 12 and is detachably fitted to the end portion of an opening side.

The end portion of the opening side in the housing portion 12 is projected from the other end portion of the outer cylinder portion body 13. In the bottle cap 1, the inner cylinder portion 10 is screwed into the opening direction with respect to the outer cylinder portion body 13, the housing portion 12 is driven, and the other end portion of the outer cylinder portion body 13 removes the bottom lid portion 16 from the opening of the housing portion 12 to drop the material housed in the housing portion 12 into the bottle 2.

Hereinafter, the bottle cap 1 according to the embodiment will be described in detail with reference to FIGS. 1 to 4. FIG. 1 shows a container 3 which is provided with the bottle cap 1 described above, and the bottle 2 on which the bottle cap 1 is mounted.

The liquid is filled in the bottle 2. The liquid to be filled is not particularly limited, however water or the like are exemplified. Water may be hot water, or may be cold water. In addition, part of the powdered raw material may be dissolved or dispersed in water in advance.

The bottle 2 has the bottle body, and the opening portion 21 which is provided on the bottle body. The bottle cap 1 is screwed into the opening portion 21.

The bottle cap 1 closes the opening portion 21 of the bottle 2.

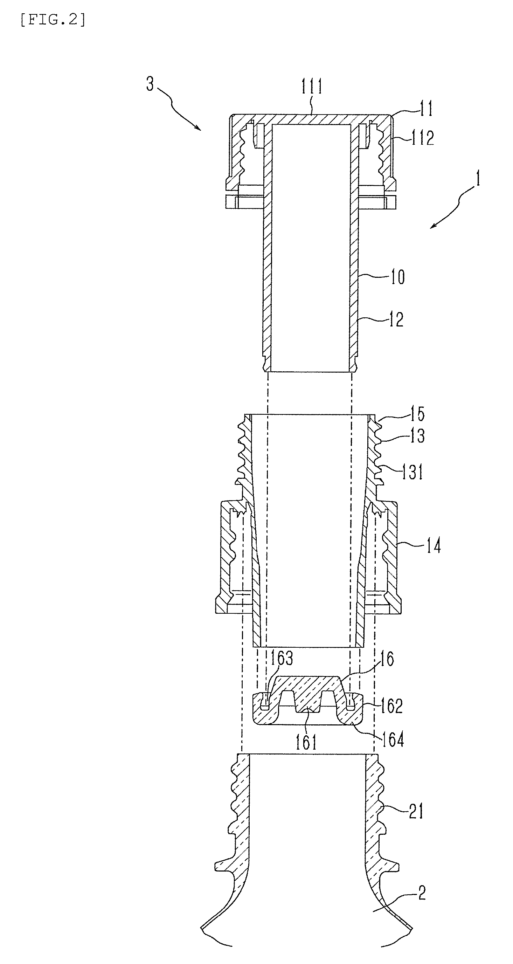

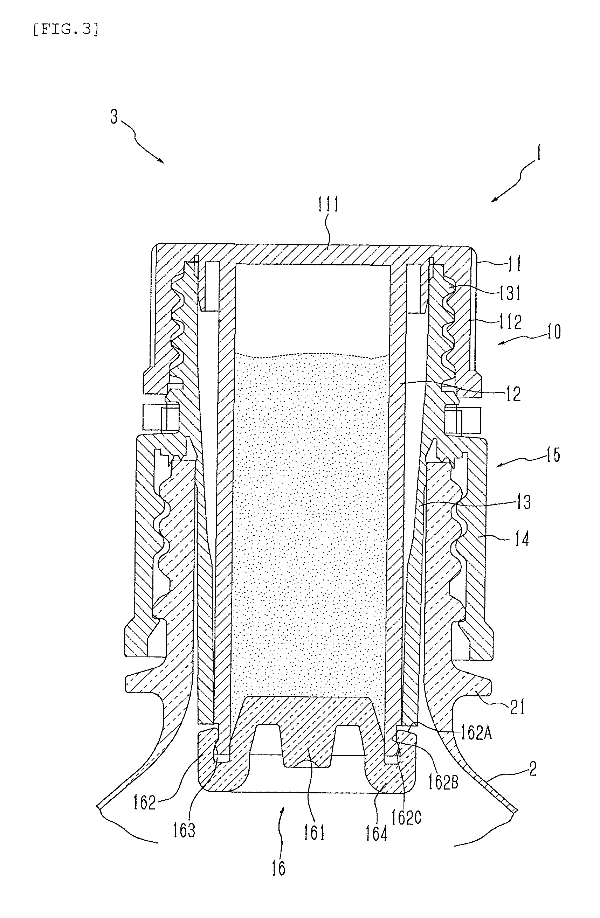

As shown in FIGS. 2 and 3, the bottle cap 1 is provided with the inner cylinder portion 10, the outer cylinder portion 15, and the bottom lid portion 16 as described above.

The inner cylinder portion 10 is provided with a lid portion 11 and the housing portion 12.

The lid portion 11 is provided with a top face portion 111 which closes the opening portion 21 of the bottle 2 and a screwing portion 112 which drops from a periphery of the top face portion 111.

The top face portion 111 has, for example, a substantially circular plane shape.

The screwing portion 112 is a peripheral wall which encloses the periphery of the top face portion 111, and a screw is engraved on an inner peripheral face of the peripheral wall.

The housing portion 12 is a cylindrical member and is inserted into the opening portion 21 of the bottle 2. The powdered raw material according to the embodiment described above is housed in the housing portion 12.

The housing portion 12 has a cylindrical shape, the one end portion thereof is fixed to a back face of the top face portion 111, and one end face is closed by the top face portion 111. The housing portion 12 and the lid portion 11 are integrally configured.

The opening is formed at the other end face of the housing portion 12, and the bottom lid portion 16 is fitted in the opening. The housing portion 12 is inserted into the outer cylinder portion body 13 of the outer cylinder portion 15, and the other end portion of the opening side of the housing portion 12 is exposed from the other end portion of the outer cylinder portion body 13.

The bottom lid portion 16 is fitted into an opening of the housing portion 12 and closes the opening of the housing portion 12. The bottom lid portion 16 is forcibly inserted in the opening of the housing portion 12 and is provided with a closing portion 161 which closes the opening, a peripheral wall portion 162 which is provided at the peripheral portion of a closing portion 161, and a fitting groove 163 which is formed between the peripheral wall portion 162 and the closing portion 161, and into which the end portion of the opening side of the housing portion 12 is forcibly inserted.

When the bottom lid portion 16 is fitted to the opening of the housing portion 12 the peripheral wall portion 162 is placed at a side direction (outside) of the end portion of the opening side of the housing portion 12, and encloses the periphery of the end portion of the opening side of the housing portion 12. An upper face 162A of the peripheral wall portion 162 faces a lower end face of the other end portion of the outer cylinder portion body 13.

The upper face 162A of the peripheral wall portion 162 facing the end portion of the outer cylinder portion body 13 is an inclined face which is inclined downward toward an outer peripheral side from an inner peripheral side of the upper face 162A.

Furthermore, an inner side face of the peripheral wall portion 162 (face facing an outer face of the housing portion 12) is configured to have a vertical face 162B and the inclined face 162C. When the bottom lid portion 16 is fitted into the opening of the housing portion 12, the vertical face 162B is substantially parallel with the outer face of the housing portion 12 and is in contact with the outer face of the housing portion 12.

The inclined face 162C is a face which is inclined downward from a lower end portion of the vertical face 162B to be remote from the outer face of the housing portion 12.

A fitting groove 163 is formed of the inner face of the peripheral wall portion 162, the outer face of the closing portion 161, the connecting portion 164 which connects the lower end portion of the peripheral wall portion 162 and the periphery of the closing portion 161.

The bottom lid portion 16 is mounted on the housing portion 12 by forcibly inserting the closing portion 161 into the opening of the housing portion 12 and fitting the end portion of the opening side of the housing portion 12 into the fitting groove 163.

The outer cylinder portion 15 is provided with the outer cylinder portion body 13 and an applying portion 14.

The outer cylinder portion body 13 is a cylindrical member without a bottom.

In the outer cylinder portion body 13, one end portion screws into the lid portion 11, and the other end portion is inserted into the opening portion 21 of the bottle 2.

Furthermore, the housing portion 12 is inserted into the inside of the outer cylinder portion body 13, and the end portion of the opening side of the housing portion 12 is projected from the other end portion of the outer cylinder portion body 13. Most of the outer peripheral face of the housing portion 12 is covered by the outer cylinder portion body 13, and only the end portion of the opening side of the housing portion 12 is exposed from the other end portion of the outer cylinder portion body 13.

Furthermore, the inner peripheral face of the outer cylinder portion body 13 is in contact with the outer peripheral face of the housing portion 12.

Furthermore, the end face of the other end portion of the outer cylinder portion body 13 faces a tip of the peripheral wall portion 162 of the bottom lid portion 16.

A screw is engraved on the outer peripheral face of the one end portion of the outer cylinder portion body 13 to form a screwing portion 131. The screwing portion 112 of the lid portion 11 is screwed to the screwing portion 131 of the outer cylinder portion body 13.

Further, when the bottle cap 1 is installed on the bottle 2, a center portion of the outer peripheral face of the outer cylinder portion body 13 is in contact with the inner wall of the opening portion 21 of the bottle 2 and is tightly sealed. In the outer peripheral face of the outer cylinder portion body 13, the center portion is continuously in contact with the inner wall of the opening portion 21 of the bottle 2 circumferentially. The center portion is a region of the other end portion side of the screwing portion 131 of the outer cylinder portion body 13, and a portion which faces the applying portion 14 to be described below.

The applying portion 14 is provided on the outer peripheral face of the outer cylinder portion body 13.

The applying portion 14 is a cylindrical member which is projected from the outer peripheral face of the outer cylinder portion body 13. In the embodiment, the applying portion 14 is provided to enclose the substantially center portion of the outer peripheral face of the outer cylinder portion body 13, and the end face on the opening portion 21 side of the bottle 2 is open.

A screw is engraved on the inner peripheral face of the applying portion 14 and is screwed in the screw which is engraved to the inner peripheral face of the opening portion of the bottle 2, and thus the bottle cap 1 is installed on the bottle 2.

In the embodiment, the applying portion 14 is formed integrally with the outer cylinder portion body 13.

Next, the method for mounting the bottle cap 1 on the bottle 2 will be described.

First, the housing portion 12 of the inner cylinder portion 10 is inserted into the outer cylinder portion body 13 of the outer cylinder portion 15. Then, the raw material is filled in the housing portion 12, and the bottom lid portion 16 is mounted thereon.

Thereafter, the outer cylinder portion body 13 of the outer cylinder portion 15 is inserted to the opening portion 21 of the bottle 2. Further, the applying portion 14 of the bottle cap 1 is screwed in the opening portion 21 of the bottle 2. Thus, the bottle cap 1 is mounted on the bottle 2. Moreover, when the applying portion 14 of the bottle cap 1 is screwed to the opening portion 21 of the bottle 2, the outer cylinder portion body 13 is in contact with the opening portion 21 of the bottle 2, and slides against the inner wall of the opening portion 21 of the bottle 2.

Subsequently, the uncapping operation of the bottle cap 1 will be described.

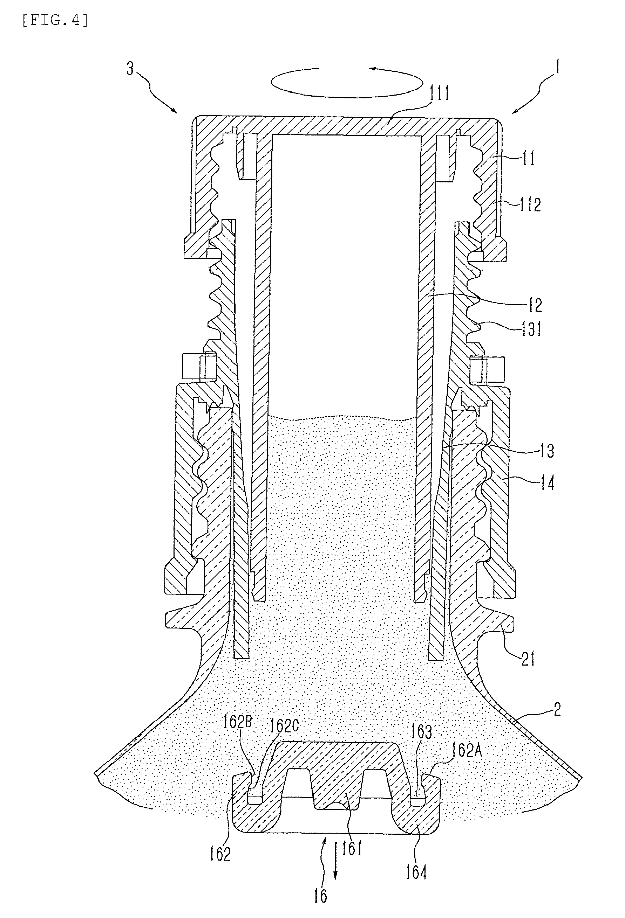

As shown in FIG. 4, the lid portion 11 is screwed to the opening direction, that is, to the upper side of FIG. 4, and thus the screwing of the lid portion 11 and the outer cylinder portion body 13 is released. Since the lid portion 11 and the housing portion 12 are integrally configured, the operation for screwing the lid portion 11 proceeds, and the housing portion 12, and further, the bottom lid portion 16 which is forcibly inserted into the housing portion 12, are driven to the upper side.

At this time, since the bottom lid portion 16 faces the end portion of the outer cylinder portion body 13 and is in contact therewith, the end face of the outer cylinder portion body 13 prevents the bottom lid portion 16 from being driven to the upper side, and by the collision of the bottom lid portion 16 with the end face of the outer cylinder portion body 13, the bottom lid portion 16 falls off from the housing portion 12.

Thus, the powdered raw material which is housed in the housing portion 12 falls into the bottle 2 and is mixed with the liquid which fills the bottle 2.

Additionally, when the lid portion 11 is screwed to the opening direction, the operation for screwing the lid portion 11 proceeds, and the housing portion 12 is slid against the inside of the outer cylinder portion body 13 toward the upper side of FIG. 4. The bottle cap 1 is opened by removing the housing portion 12 from the inside of the outer cylinder portion body 13.

The embodiments of the present invention described above are examples of the present invention, and it is also possible to adopt various configurations other than the above. For example, the mixture of two or more kinds of the powdered raw material may be used, and materials other than the powdered raw material may be included.

[EXAMPLES]

Hereinafter, the embodiment will be specifically described using examples and comparative examples, but the embodiment is not limited thereto.

(Method for Preparation of the Powdered Raw Material)

First, tea leaves of green tea obtained using a commonly used method were pulverized by a jet mill and were classified. Here, powdered raw materials shown in Table 1 were prepared by changing the conditions of the jet mill, respectively.

(Evaluation Items)

The obtained powder was passed through a wire mesh of 60 Mesh, and the following evaluations were performed using the powder from which coarse particles were removed. Moreover, the measurements were performed three times to reduce effects of measurement variation with respect to each evaluation criteria, and averages of the three measured values were used.

<Bulk Density Measurement>

A loose bulk density and a compact bulk density were measured using a multi-functional powder property measuring instrument multi-tester (model: MT-1001, manufactured by Seishin Enterprise Co.). In measurement, the powder was dropped to a cell container of 100 cm$^3$ through a funnel to be filled, an overflowed portion was removed, and the loose bulk density was calculated from a mass of filled powder and a volume of the cell container.

In the same manner, the powder was dropped to a cell container of 100 cm$^3$ through a funnel to be filled, tapping was performed 180 times with a tapping stroke of 18 mm at a tapping speed of 1 time/second, an overflowed portion was removed, and the compact bulk density was calculated from the mass of filled powder and the volume of the cell container.

<Degree of Compaction and Uniformity>

A degree of compaction and a uniformity were calculated by the following formulae (2) and (3), respectively.

$$\text{degree of compaction [\%]} = (\text{compact bulk density} - \text{loose bulk density}) / \text{compact bulk density} \times 100 \quad (2)$$

$$\text{uniformity [-]} = (\text{particle diameter } D_{60}) / (\text{particle diameter } D_{10}) \quad (3)$$

<Particle Size Distribution>

A particle size distribution was measured, specifically, as follows. After adjusting to be 2% by mass by adding the powdered raw material into ethanol, an ultrasonic wave was applied to disperse for 1 minute, and the dispersed dispersion was used as a measurement sample. The particle size distribution was measured in an absorbance range of 0.1 to 0.2 (wave length 680 nm, optical path length 6 mm, quartz cell) using a laser diffraction-scattering particle size distribution analyzer (SK laser micron sizer LMS-24, manufactured by Seishin Enterprise Co.)

<Degree of Dispersion>

A degree of dispersion was measured using the multi-functional powder property measuring instrument multi-tester (model: MT-1001, manufactured by Seishin Enterprise Co.). First, a mass of a watch glass was measured and set in a sample collection box. Then, the 10 g sample was set to a sample dropping unit. Next, a shutter of a hopper portion was opened, and the powdered raw material was put in the watch glass. The mass of the sample put in the watch glass was measured, and the degree of dispersion was calculated by the following formula (1).

$$\text{degree of dispersion [\%]} = \text{(dropped mass - the mass on the watch glass)/dropped mass} \times 100 \quad (1)$$

<Fillability Test in the Bottle Cap>

1.0 g of each powdered raw material described in Table 1 was dropped to the bottle cap 1 from the height of 5 cm at a stretch, respectively. When the amount which was scattered to the outside of the bottle cap 1 was equal to or more than 0.1 g, it was evaluated as "X", and when the amount was less than 0.1 g, it was evaluated as "O".

<Dispersibility in Water>

First, the bottle cap 1 which was filled with the powdered raw material 1.0 g was installed on a bottle which was filled with 200 mL water of temperature 25°C. Next, the powdered raw material was released into the bottle by performing an uncapping operation. Finally, stirring was performed at 60 rpm for 10 seconds using a shaker of vertically reciprocating type, and then a dispersibility of the powdered raw material was observed. When lumps were visually observed after stirring, it was evaluated as "X", and when the lumps were not visually observed, it was evaluated as "O".

The above evaluation results were summarized in Table 2. The fillability in the bottle cap 1 of the powdered raw material in Examples 1 to 6 and Comparative Example 1 was excellent. On the other hand, the scattered amount of the powdered raw material in comparative example 2 to the outside of the bottle cap 1 was large, and it was difficult to use in filling the bottle cap 1.

Furthermore, lumps were not observed in the powdered raw material in Examples 1 to 6 after stirring, and the dispersibility was excellent. On the other hand, lumps were observed in the powdered raw material which was prepared in Comparative Example 1 after stirring. Furthermore, the drinking feeling of green tea beverages in Examples 1 to 6 was excellent, however the sensation felt on the tongue and the throat of the powdered raw material in Comparative Example 1 was bad due to the lumps, and the drinking feeling was bad.

[Table 1]

| | Particle diameter [μm] | | | | Loose bulk density [g/cm³] | Compact bulk density [g/cm³] | Degree of compaction [%] | Uniformity [-] | Degree of dispersion [%] |
|---|---|---|---|---|---|---|---|---|---|
| | $D_{10}$ | $D_{50}$ | $D_{90}$ | Standard deviation σ | | | | | |
| Example 1 | 2.8 | 6.7 | 12.5 | 5.6 | 0.17 | 0.36 | 43 | 2.8 | 17 |
| Example 2 | 3.1 | 8.3 | 19.0 | 7.4 | 0.19 | 0.40 | 44 | 3.2 | 16 |
| Example 3 | 2.8 | 7.8 | 19.6 | 7.3 | 0.22 | 0.44 | 43 | 3.4 | 9 |
| Example 4 | 2.9 | 7.8 | 18.5 | 7.3 | 0.22 | 0.42 | 45 | 3.2 | 10 |
| Example 5 | 3.1 | 9.6 | 26.1 | 8.3 | 0.25 | 0.50 | 49 | 3.9 | 22 |
| Example 6 | 3.1 | 10.1 | 31.7 | 9.2 | 0.28 | 0.52 | 46 | 4.2 | 14 |
| Comparative example 1 | 3.0 | 7.5 | 24.4 | 7.9 | 0.26 | 0.53 | 46 | 3.1 | 4 |
| Comparative example 2 | 3.2 | 9.8 | 28.5 | 9.4 | 0.26 | 0.53 | 45 | 3.9 | 42 |

[Table 2]

|  | Dispersibility | Fillability |
|---|---|---|
| Example 1 | O | O |
| Example 2 | O | O |
| Example 3 | O | O |
| Example 4 | O | O |
| Example 5 | O | O |
| Example 6 | O | O |
| Comparative Example 1 | X | O |
| Comparative Example 2 | - | X |

Moreover, a test of fillability into the bottle cap and a test of dispersibility in water of lactic acid bacteria powder retaining activity by processing lactic acid bacteria using a representative cryogenic pulverization, powdered yeast and vitamins were performed, respectively after adjusting the particle size distribution in the same manner as the above-described tea leaves of green tea.

As a result, the powdered raw material of which the degree of dispersion is equal to or lower than 40% had an excellent filling ability in the bottle cap 1. On the other hand, the powdered raw material of which the degree of dispersion is higher than 40% had a large amount scattered to the outside of the bottle cap 1, and it was difficult to use in filling the bottle cap 1.

Furthermore, the lumps were not observed in the powdered raw material of which the degree of dispersion is equal to or higher than 5% after stirring, and the dispersibility was excellent. On the other hand, the lumps were observed in the powdered raw material of which the degree of dispersion is equal to or lower than 5% after stirring.

Furthermore, the drinking feeling of the beverage which used the powdered raw material of which the degree of dispersion is equal to or higher than 5% was excellent, however the sensation felt on the tongue and the throat of the beverage which used the powdered raw material of which the degree of dispersion is lower than 5% was bad, and the drinking feeling was bad due to the lumps.

## Claims

1. A use of a powdered raw material in a housing portion (12) of a type of a bottle cap which has the housing portion that houses the powdered raw material, and of which the housing portion can be opened by an uncapping operation and then causes the raw material to be released into the bottle (2) ,
   wherein using a multi-functional powder property measuring instrument multi-tester, when the powdered raw material is dropped onto a watch glass from a powder falling point, and a dropped mass and a mass on the watch glass are measured, a degree of dispersion which is obtained from the following formula (1) is equal to or higher than 5% and equal to or lower than 40%:

$$\text{degree of dispersion [\%]} = (\text{dropped mass} - \text{mass on the watch glass}) / \text{the dropped mass} \times 100 \quad (1),$$

   wherein the multi-functional powder property measuring instrument multi-tester is MT-1001 by Seishin Enterprise Co.

2. The use of a powdered raw material according to claim 1,
   wherein a degree of compaction which is calculated by the following formula (2) is equal to or higher than 30% and equal to or lower than 60%:

$$\text{degree of compaction [\%]} = (\text{compact bulk density} - \text{loose bulk density}) / \text{compact bulk density} \times 100 \quad (2).$$

**3.** The use of a powdered raw material according to claim 1 or 2,
wherein a particle diameter $D_{50}$ is equal to or higher than 3 $\mu$m and equal to or lower than 15 $\mu$m,
wherein $D_x$ represents a particle diameter of which a cumulative mass distribution becomes X% in a particle size distribution measured using a laser diffraction-scattering particle size distribution analyzer.

**4.** The use of a powdered raw material according to claim 3,
wherein a uniformity which is defined by the following formula: (particle diameter $D_{60}$) / (particle diameter $D_{10}$) is equal to or lower than 5.

**5.** The use of a powdered raw material according to claim 3 or 4,
wherein a particle diameter $D_{10}$ is equal to or higher than 1 $\mu$m.

**6.** The use of a powdered raw material according to any one of claims 3 to 5,
wherein a particle diameter $D_{90}$ is equal to or lower than 50 $\mu$m.

**7.** The use of a powdered raw material according to any one of claims 3 to 6,
wherein a standard deviation sigma of the particle size distribution is equal to or lower than 15 $\mu$m.

**8.** The use of a powdered raw material according to any one of claims 1 to 7,
wherein a loose bulk density is equal to or higher than 0.1 g/cm$^3$ and equal to or lower than 0.4 g/cm$^3$.

**9.** The use of a powdered raw material according to any one of claims 1 to 8,
wherein a compact bulk density is equal to or higher than 0.2 g/cm$^3$ and equal to or lower than 0.8 g/cm$^3$.

**10.** The use of a powdered raw material according to any one of claims 1 to 9, comprising:

one or more kinds of supplements selected from one group of vitamins, minerals, amino acids, fatty acids, dietary fibers, sugars, proteins, turmerics, and polyphenols.

**11.** The use of a powdered raw material according to claim 10,
wherein the supplement is vitamins.

**12.** The use of a powdered raw material according to any one of claims 1 to 9 which is used in foods and beverages or in pharmaceuticals or in cosmetics.

**13.** A bottle cap comprising:

an inner cylinder portion (10) having a cylindrical housing portion (12) of which one end face is closed and the other end face is open and which is inserted into an opening portion (21) of a bottle (2); an outer cylinder portion (15) having an outer cylinder portion body (13) into which the housing portion is inserted, of which one end portion is screwed into the inner cylinder portion and the other end portion is inserted into the opening portion of the bottle and which is screwed into the opening portion of the bottle; and
a bottom lid portion (16) which closes the opening of the housing portion and is detachably fitted to an end portion of an opening side,
wherein the end portion of the opening side of the housing portion is projected from the other end portion of the outer cylinder portion body,
wherein the housing portion is driven by screwing the inner cylinder portion to an opening direction with respect to the outer cylinder portion body, the bottom lid portion is pulled out from the opening of the housing portion by the other end portion of the outer cylinder portion body, and thus, a housed material in the housing portion is dropped into the bottle,
wherein the housed material comprises powdered raw material,
wherein using a multi-functional powder property measuring instrument multi-tester, when the powdered raw material is dropped onto a watch glass from a powder falling point, and a dropped mass and a mass on the watch glass are measured, a degree of dispersion which is obtained from the following formula (1) is equal to or higher than 5% and equal to or lower than 40%:

```
degree of dispersion [%] = (dropped mass - mass on the
watch glass) / the dropped mass × 100     (1),
```

wherein the multi-functional powder property measuring instrument multi-tester is MT-1001 by Seishin Enterprise Co.

**14.** A container, comprising:

the bottle cap according to claim 13; and
the bottle to which the bottle cap is mounted.


**Patentansprüche**

**1.** Verwendung eines pulverförmigen Rohmaterials in einem Gehäuseabschnitt (12) nach Art einer Flaschenverschlusskappe, die den Gehäuseabschnitt, der das pulverförmige Rohmaterial aufnimmt, aufweist, und von welcher der Gehäuseabschnitt durch einen Öffnungsvorgang geöffnet werden kann und dann bewirkt, das Rohmaterial in die Flasche (2) freizugegeben,
wobei, wenn das pulverförmige Rohmaterial von einer Pulverfallstelle auf ein Sichtfenster fallengelassen wird, unter Verwendung eines multi-funktionalen Pulvereigenschaftsmessinstrument-Multitesters, eine fallengelassene Masse und eine Masse auf dem Sichtfenster gemessen werden, und ein Verteilungsgrad, der durch die folgende Formel (1) ermittelt wird, gleich oder höher als 5% und gleich oder kleiner als 40% ist:

```
Verteilungsgrad [%] = (fallengelassene Masse – Masse
auf dem Sichtfenster) / fallengelassene Masse x 100 (1),
```

wobei der multi-funktionale Pulvereigenschaftsmessinstrument-Multitester ein MT-1001 von Seishin Enterprise Co. ist.

**2.** Verwendung eines pulverförmigen Rohmaterials nach Anspruch 1,
wobei ein Verdichtungsgrad, der durch die folgende Formel (2) berechnet wird, gleich oder höher als 30% und gleich oder kleiner als 60% ist:

```
Verdichtungsgrad [%] = (verdichtete Schüttdichte –
lockere Schüttdichte) / verdichtete Schüttdichte x 100
(2).
```

**3.** Verwendung eines pulverförmigen Rohmaterials nach Anspruch 1 oder 2,
wobei ein Partikeldurchmesser $D_{50}$ gleich oder höher als 3 $\mu$m und gleich oder geringer als 15 $\mu$m ist,
wobei $D_x$ einen Partikeldurchmesser darstellt, bei dem eine kumulative Massenverteilung bei einer Partikelgrößenverteilung, die unter Verwendung eines Laserbeugungs-Streuungspartikelgrößenverteilungsanalysegeräts gemessen wird, X% wird.

**4.** Verwendung eines pulverförmigen Rohmaterials nach Anspruch 3,
wobei eine Einheitlichkeit, die durch die folgende Formel definiert ist, gleich oder kleiner als 5 ist:

```
(Partikeldurchmesser D₆₀) / (Partikeldurchmesser D₁₀).
```

**5.** Verwendung eines pulverförmigen Rohmaterials nach Anspruch 3 oder 4,
wobei ein Partikeldurchmesser $D_{10}$ gleich oder größer als 1 $\mu$m ist.

**6.** Verwendung eines pulverförmigen Rohmaterials nach einem der Ansprüche 3 bis 5,
wobei ein Partikeldurchmesser $D_{90}$ gleich oder kleiner als 50 $\mu$m ist.

**7.** Verwendung eines pulverförmigen Rohmaterials nach einem der Ansprüche 3 bis 6,
wobei eine Standardabweichung Sigma der Partikelgrößenverteilung gleich oder kleiner als 15 $\mu$m ist.

**8.** Verwendung eines pulverförmigen Rohmaterials nach einem der Ansprüche 1 bis 7,
wobei eine lockere Schüttdichte gleich oder größer als 0.1 g/cm$^3$ und gleich oder kleiner als 0.4 g/cm$^3$ ist.

**9.** Verwendung eines pulverförmigen Rohmaterials nach einem der Ansprüche 1 bis 8,
wobei eine verdichtete Schüttdichte gleich oder größer als 0.2 g/cm$^3$ und gleich oder kleiner als 0.8 g/cm$^3$ ist.

**10.** Verwendung eines pulverförmigen Rohmaterials nach einem der Ansprüche 1 bis 9, aufweisend:

eine oder mehrere Arten von Nahrungsergänzungsmitteln, die aus einer Gruppe von Vitaminen, Mineralstoffen, Aminosäuren, Fettsäuren, diätischen Fasern, Zucker, Proteinen, Kurkuma, und Polyphenolen ausgewählt sind.

**11.** Verwendung eines pulverförmigen Rohmaterials nach Anspruch 10,
wobei das Nahrungsergänzungsmittel Vitamine sind.

**12.** Verwendung eines pulverförmigen Rohmaterials nach einem der Ansprüche 1 bis 9, das bei Nahrungsmitteln und Getränken oder bei Pharmazeutika oder bei Kosmetika verwendet wird.

**13.** Flaschenverschlusskappe, aufweisend:

ein inneres Zylinderteil (10), das einen zylindrischen Gehäuseabschnitt (12) aufweist, von dem ein Seitenende geschlossen und das andere Seitenende offen ist und das in einen Öffnungsabschnitt (21) einer Flasche (2) eingesetzt ist;
ein äußeres Zylinderteil (15), das einen äußeren Zylindergehäuseabschnitt (13) aufweist, in den der Gehäuseabschnitt eingesetzt ist, von dem ein Endabschnitt in das innere Zylinderteil eingeschraubt und der andere Endabschnitt in den Öffnungsabschnitt der Flasche eingesetzt ist und der in den Öffnungsabschnitt der Flasche eingeschraubt ist; und
ein Deckelbodenteil (16), das die Öffnung des Gehäuseabschnitts verschließt und abnehmbar an einem Endabschnitt der Öffnungsseite angebracht ist,
wobei der Endabschnitt der Öffnungsseite des Gehäuseabschnitts von dem anderen Endabschnitt des äußeren Zylindergehäuseabschnitts hervorsteht,
wobei der Gehäuseabschnitt durch Schrauben des inneren Zylinderteils in eine Öffnungsrichtung in Bezug auf den äußeren Zylindergehäuseabschnitt getrieben wird, wobei das Deckelbodenteil aus der Öffnung des Gehäuseabschnitts durch den anderen Endabschnitt des äußeren Zylindergehäuseabschnitts gezogen wird, und somit das in dem Gehäuseabschnitt aufgenommene Material in die Flasche fallengelassen wird,
wobei das aufgenommene Material pulverförmiges Rohmaterial aufweist,
wobei, wenn das pulverförmige Rohmaterial von einer Pulverfallstelle auf ein Sichtfenster fallengelassen wird, unter Verwendung eines multi-funktionalen Pulvereigenschaftsmessinstrument-Multitesters, eine fallengelassene Masse und eine Masse auf dem Sichtfenster gemessen werden, und ein Verteilungsgrad, der durch die folgende Formel (1) ermittelt wird, gleich oder höher als 5% und gleich oder kleiner als 40% ist:

```
Verteilungsgrad [%] = (fallengelassene Masse - Masse
auf dem Sichtfenster) / fallengelassene Masse x 100 (1),
```

wobei der multi-funktionale Pulvereigenschaftsmessinstrument-Multitester ein MT-1001 von Seishin Enterprise Co. ist.

**14.** Behälter, aufweisend:

die Flaschenverschlusskappe nach Anspruch 13; und
die Flasche, an welcher die Flaschenverschlusskappe angebracht ist.

**Revendications**

1.  Utilisation d'une matière première pulvérulente dans une partie de logement (12) d'un type de bouchon de flacon qui présente la partie de logement qui reçoit la matière première pulvérulente, et dont la partie de logement peut être ouverte par une opération d'enlèvement du bouchon et provoque ensuite la libération de la matière première pulvérulente dans le flacon (2),
    dans laquelle l'utilisation d'un instrument multi-testeur multifonctionnel de mesure de propriété de poudre, lorsque la matière première pulvérulente tombe sur un verre de montre à partir d'un point de chute de poudre, et une masse tombée et une masse sur le verre de montre sont mesurées, un degré de dispersion qui est obtenu par la formule suivante (1) est supérieur ou égal à 5% et inférieur ou égal à 40% :

    ```
    degré de dispersion [%] = (masse tombée - masse sur le
    verre de montre) / masse tombée x 100    (1),
    ```

    dans laquelle l'instrument multi-testeur multifonctionnel de mesure de propriété de poudre est MT-1001 de Seishin Enterprise Co.

2.  Utilisation d'une matière première pulvérulente selon la revendication 1,
    dans laquelle un degré de compactage qui est calculé par la formule suivante (2) est supérieur ou égal à 30% et inférieur ou égal à 60% :

    ```
    degré de compactage [%] = (densité apparente compacte
    - densité apparente non tassée) / densité apparente
    compacte x 100    (2).
    ```

3.  Utilisation d'une matière première pulvérulente selon la revendication 1 ou 2,
    dans laquelle un diamètre de particule $D_{50}$ est supérieur ou égal à 3 $\mu$m et inférieur ou égal à 15 $\mu$m,
    dans laquelle $D_x$ représente un diamètre de particule dont une distribution en masse cumulée devient X% dans une distribution de taille de particules mesurée en utilisant un analyseur de distribution de taille de particules par diffraction-diffusion laser.

4.  Utilisation d'une matière première pulvérulente selon la revendication 3,
    dans laquelle une uniformité est définie par la formule suivante : (diamètre de particule $D_{60}$) / (diamètre de particule $D_{10}$) est inférieure ou égale à 5.

5.  Utilisation d'une matière première pulvérulente selon la revendication 3 ou 4,
    dans laquelle un diamètre de particule $D_{10}$ est supérieur ou égal à 1 $\mu$m.

6.  Utilisation d'une matière première pulvérulente selon l'une quelconque des revendications 3 à 5,
    dans laquelle un diamètre de particule $D_{90}$ est inférieur ou égal à 50 $\mu$m.

7.  Utilisation d'une matière première pulvérulente selon l'une quelconque des revendications 3 à 6,
    dans laquelle un écart-type sigma de la distribution de taille de particule est inferieur ou égal à 15 $\mu$m.

8.  Utilisation d'une matière première pulvérulente selon l'une quelconque des revendications 1 à 7,
    dans laquelle une densité apparente non tassée est supérieure ou égale à 0,1 g/cm$^3$ et inférieure ou égale à 0,4 g/cm$^3$.

9.  Utilisation d'une matière première pulvérulente selon l'une quelconque des revendications 1 à 8,
    dans laquelle une densité apparente compacte est supérieure ou égale à 0,2 g/cm$^3$ et inférieure ou égale à 0, 8 g/cm$^3$.

**10.** Utilisation d'une matière première pulvérulente selon l'une quelconque des revendications 1 à 9, comprenant :

un ou plusieurs type(s) de compléments choisis dans un groupe de vitamines, de minéraux, d'acides aminés, d'acides gras, de fibres alimentaires, de sucres, de protéines, de curcuma et de polyphénols.

**11.** Utilisation d'une matière première pulvérulente selon la revendication 10, dans laquelle le complément représente des vitamines.

**12.** Utilisation d'une matière première pulvérulente selon l'une quelconque des revendications 1 à 9, qui est utilisée dans des aliments et boissons ou dans des produits pharmaceutiques ou dans des produits cosmétiques.

**13.** Bouchon de flacon comprenant :

une partie de cylindre interne (10) ayant une partie de logement cylindrique (12) dont une face d'extrémité est fermée et l'autre face d'extrémité est ouverte et qui est insérée dans une partie d'ouverture (21) d'un flacon (2) ;
une partie de cylindre externe (15) ayant un corps de partie de cylindre externe (13) dans lequel la partie de logement est insérée, dont une partie d'extrémité est vissée dans la partie de cylindre interne et l'autre partie d'extrémité est insérée dans la partie d'ouverture du flacon et qui est vissée dans la partie d'ouverture du flacon ; et
une partie inférieure de couvercle (16) qui ferme l'ouverture de la partie de logement et est ajustée de manière amovible à une partie d'extrémité d'un côté d'ouverture,
où la partie d'extrémité du côté d'ouverture de la partie de logement fait saillie à partir de l'autre partie d'extrémité du corps de partie de cylindre externe,
où la partie de logement est entraînée par vissage de la partie de cylindre interne dans une direction d'ouverture par rapport au corps de partie de cylindre externe, la partie inférieure de couvercle est retirée de l'ouverture de la partie de logement par l'autre partie d'extrémité du corps de partie de cylindre externe, et par conséquent, un matériau reçu dans la partie de logement tombe dans le flacon,
où le matériau reçu comprend une matière première pulvérulente,
où l'utilisation d'un instrument multi-testeur multifonctionnel de mesure de propriété de poudre, lorsque la matière première pulvérulente tombe sur un verre de montre à partir d'un point de chute de poudre, et une masse tombée et une masse sur le verre de montre sont mesurées, un degré de dispersion qui est obtenu par la formule suivante (1) est supérieur ou égal à 5% et inférieur ou égal à 40% :

$$\text{degré de dispersion [\%]} = (\text{masse tombée - masse sur le verre de montre}) / \text{masse tombée} \times 100 \quad (1),$$

où l'instrument multi-testeur multifonctionnel de mesure de propriété de poudre est MT-1001 de Seishin Enterprise Co.

**14.** Récipient, comprenant :

le bouchon de flacon selon la revendication 13 ; et
le flacon auquel le bouchon de flacon est monté.

[FIG.1]

[FIG.2]

[FIG.3]

[FIG.4]

**EP 2 702 878 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003034367 A

- JP 2006296341 A